# EUROPEAN PATENT SPECIFICATION

(11) **EP 1 644 381 B1**
(45) Date of publication and mention of the grant of the patent: **17.03.2010**
(21) Application number: 04743729.8
(22) Date of filing: 30.06.2004
(51) Int. Cl.: C07D 495/04

(54) **PROCESS FOR THE PREPARATION OF CRYSTALLINE POLYMORPH OF A PLATELET AGGREGATION INHIBITOR DRUG**
PROZESS ZUR HERSTELLUNG EINES KRISTALLINEN POLYMORPHS EINES DIE PLÄTTCHEN AGGREGATION HEMMENDEN ARZNEISTOFFS
PROCEDE DE PREPARATION D'UN POLYMORPHE CRISTALLIN D'UN MEDICAMENT INHIBITEUR DE L'AGREGATION DES PLAQUETTES

(30) Priority: 02.07.2003 HU 0302028; 23.06.2004 HU 0401272
(43) Date of publication of application: 12.04.2006
(73) Proprietor: EGIS Gyógyszergyár Nyilvánosan Müködõ Részvénytársaság, 1106 Budapest (HU)
(72) Inventor: KOTAY NAGY, Péter, H-2600 Vác (HU); SIMIG, Gyula, H-1126 Budapest (HU); BARKOCZY, József, H-1016 Budapest (HU); GREGOR, Tamás, H-2141 Csömör (HU); FARKAS, Béla, H-8200 Veszprém (HU); VERECZKEYNÉ DONÁTH, Györgyi, H-1034 Budapest (HU); NAGY, Kálmán, H-1025 Budapest (HU); KÖRTVELYESSY, Gyuláné, H-1022 Budapest (HU); SZENT-KIRALLYI, Zsuzsanna, H-1223 Budapest (HU)
(74) Representative: Hirsch & Associés
(86) International application number: PCT/HU2004/000070
(87) International publication number: WO 2005/003139

(56) References cited:
- EP-A- 0 281 459
- WO-A-99/65915
- WO-A-03/051362
- CAIRA M R: "CRYSTALLINE POLYMORPHISM OF ORGANIC COMPOUNDS" 1998, TOPICS IN CURRENT CHEMISTRY, SPRINGER, BERLIN, DE, PAGE(S) 163-208 , XP001156954 ISSN: 0340-1022 the whole document

## Description

The present invention relates to a new method for the preparation of the polymorph form 1 of methyl (*S*) (+)-(2-chlorophenyl)-2-(6,7-dihydro-4*H*-thieno [3,2-c]pyridine-5yl-acetate hydrogensulfate of the Formula

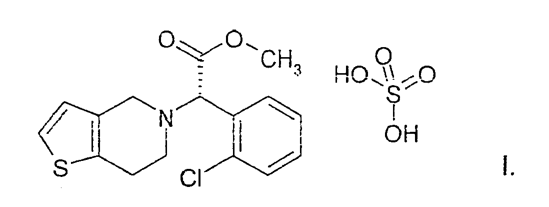

### TECHNICAL BACKGROUND OF THE INVENTION

(*S*)-(+)-(2-chlorophenyl)-2-(6,7-dihydro-4*H*-thieno [3,2-c]pyridine-5-yl-acetate hydrogensulfate, is a known platelet aggregation inhibitor drug, having INN clopidogrel hydrogensulfate.

Clopidogrel hydrogensulfate is described first in European Patent Specification No. 281 459. Hungarian equivalent of this patent is Hungarian Patent No. 197 909.
The product is characterized by its melting point and optical rotation, which are 182°C and [α]_{D}²⁰= +51.61 (c = 2.044 g/100 ml, methanol) respectively. Crystal form of the product is not mentioned.
Polymorph forms of clopidogrel hydrogensulfate are described first in French Patent Application No. 98/07464. Polymorph form 1 is specified as monocline crystal form, characterized by X-ray diffraction pattern and infrared spectrum.

### Melting point and optical rotation of polymorph form 1 are

184°C and [α]_{D} ²⁰= +55.1° (c = 1.891 / 100 ml, methanol) respectively. On the basis of these data, the authors state that the polymorph form described in the European patent specification No. 281 459 is polymorph form 1. The orthorhombic polymorph form 2 is characterized by its melting point of 176°C in the specification of French Patent Application No. 98/07464.
According to the cited specification, polymorph form 1 is prepared by adding 80% sulfuric acid to a solution of clopidogrel base in acetone in equimolar amount at 20 °C. The solvent is evaporated partly, the residue is cooled to 0-5°C and the precipitate is filtered.

Polymorph form 2 is precipitated out of the filtrate resulting from the process of the preparation of polymorph form 1, which solution is stored below 40 °C for 3-6 months.

According to the above mentioned patent specification, polymorph form 2 can also be prepared by dissolving clopidogrel base in acetone, then adding 80% sulfuric acid in an equimolar amount at 20 °C, without or in the presence of seeding crystals. The reaction mixture is boiled for two hours, then the solvent is evaporated partly, the residue is either cooled to -5°C, and the precipitated product is filtered, or seeding crystals are added, the reaction mixture is stirred at 20°C then filtered.

According to the specification of International Patent Application No. 02/059128, polymorph form 1 of clopiodogrel hydrogensulfate is prepared also by the reaction of a solution of clopidogrel base in threefold amount of acetone calculated on the amount of clopidogrel base with concentrated sulfuric acid between 0-5 °C. After addition of sulfuric acid, one more part of acetone is added, then the reaction mixture is stirred for 4 hours. Subsequently the polymorph form 1 is isolated with a melting point of 185°C.

In the specification of the International Patent Application No. 03/051362, different polymorph forms of clopidogrel hydrogensulfate are obtained by recrystallising clopidogrel hydrogensulfate from different solvents or by the precipitation with anti-solvents from its solutions.

The different polymorphs are assigned with roman numerals, wherein I. corresponds to the polymorph form 1 and II. to the polymorph form 2 of the present invention.

According to the specification of the Patent Application above, the recrystallisation of clopidogrel hydrogensulfate from different solvents results in the formation of the thermodinamically controlled polymorph form 2, except when 2-propanol is used. In this case polymorph form IV is formed.

Different results are obtained if a solution of clopidogrel hydrogensulfate is evaporated to dryness and the residue is triturated with another solvent, or a different solvent in which solubility of clopidogrel is poor - so-called anti-solvent - is added to the solution of the clopidogrel hydrogensulfate, thus reducing its solubility in the obtained mixture, resulting in the precipitation of clopidogrel hydrogensulfate.

Different polymorph forms are produced using different solvent/anti-solvent pairs.

According to International Patent Application No. 03/051362, polymorph form 2 is formed by adding diethyl ether to the solution of clopidogrel hydrogensulfate in acetonitrile.
Using methanol or acetone as solvent in similar processes and an ether-type solvent as anti-solvent, the product can be either polymorph form 1 or amorphous form of clopidogrel hydrogensulfate.

To obtain polymorph form 1 product, all processes described in the said Patent Application use an ether-type anti-solvent to precipitate out of the clopidogrel hydrogen sulfate solution or to triturate the evaporated residue.

According to the examples above, it is impossible to predict which polymorph form will be precipitated or converted into another form during the interaction of clopidogrel hydrogensulfate and a selected solvent.

Pharmacopoeias pose high requirements towards the purity and morphological uniformity of pharmaceutical active ingredients. Such requirements are justified by the fact that absorption of different polymorph forms may be different in vivo.

In an earlier period, clopidogrel hydrogensulfate tablets contained polymorph form 1.

Properties of different polymorph forms may have different properties from the pharmaceutical technology point of view as well. Morphologically uniform products have constant filtration and delivery properties, which make easier to comply the quality of the products with high requirements.

More controllable technology is advantageous from economic point of view and of the preparation of the active ingredient and the composition as well.

### FIELD OF THE INVENTION

There is a long-felt need for the industrially applicable and reproducible preparation process of morphologically uniform and pure clopidogrel hydrogensulfate, which complies with the requirements of Pharmacopoeias.

Our experience demonstrate that the preparation of polymorph form 1 can not be accomplished acceptably neither by the reproduction of the process according to the specification of French Patent Application No 98/07464, nor the processes described in International Patent Application No. 02/059128.

International Patent Application WO03/051362 describes several processes to produce the desired polymorph form 1, but only a few solvents are suggested to be used in these processes. Moreover, these processes result in a mixture of amorphous form and polymorph form 1 in some cases. This is undesirable, because it is preferable to handling with the morphologically uniform products in the technology.

Our aim is to provide a new process, which allows the use of different solvent types to produce clopidogrel hydrogensulfate in a morphologically uniform polymorph form 1. In this case, solvents can be selected according to the requirement of the manufacturing process.

### SUMMARY OF THE INVENTION

The present invention relates to a new process for the preparation of the polymorph form 1 of methyl (*S*)-(+)-(2-chlorophenyl)-2-(6,7-dihydro-4*H*-thieno[3,2-c]pyridine-5-yl-acetate hydrogensulfate of the formula (I) which comprise
a.) dissolving clopidogrel base in an "A" type solvent, adding sulfuric acid or a mixture of sulfuric acid and an "A" or "B" type solvent to the mixture, adding the obtained mixture containing clopidogrel hydrogensulfate to a mixture of a "B" type solvent containing clopidogel hydrogensulfate polymorph form as a suspension,
   or
b.) dissolving clopidogrel base in a mixture of "A" and "B" type solvents, adding clopidogrel hydrogensulfate polymorph form 1 to the solution, then adding sulfuric acid or a mixture of sulfuric acid with an "A" or "B" type solvent to the obtained mixture,
   and subsequently filtering, optionally washing and drying the formed precipitate.

The basis of our invention is the recognition that changing the polarity of the solution containing clopidogrel hydrogensulfate by using other suitable solvent in the presence of the polymorph form 1 results in the formation of polymorph form 1. The obtained precipitate is neither the expected amorphous form, nor the thermodinamically controlled more stable polymorph form 2, but the unexpected polymorph form 1.

This is very surprising, because our experiments prove, that the formation of thermodinamically more stable polymorph 2 is so favourable, that in the reaction of a solution containing clopidogrel base with sulfuric acid, polymorph form 2 is formed, even in the presence of the clopidogrel hydrogensulfate polymorph form 1.

It is known that different polymorph forms are precipitated out of the solutions containing clopidogrel hydrogensulfate according to the solvents used. It is surprising that the technical solution we found for the preparation of clopidogrel hydrogensulfate polymorph form 1 is reproducible and industrially applicable process using different types of solvents.

### DETAILED DESCRIPTION OF THE INVENTION

According to the present invention, halogenated solvents, preferably aliphatic halogenated solvents, more preferably dichloromethane; ketones, more preferably acetone; 2-propanol or protic solvents can be used as, A" type solvents in both variants of the process. According to the process of the present invention apolar solvents and ester type solvents, preferably ethyl acetate can be used as "B" type solvents. Ethers or saturated hydrocarbons are used as apolar solvents, Diethyl ether, tetrahydrofurane, diisopropyl ether, peferably diisopropyl ether can be used as ether. Hexane, cyclohexane or heptane can be used as saturated hydrocarbon.

It was found that the preparation of the polymorph form 1 according to the known processes proceed with difficulties. The examples of the specification of the International Patent Application No 99/65915 demonstrate to the person skilled in the art, that the use of seeding crystal of the polymorph form2 is advantageous for the preparation of the polymorph form 2, otherwise, polymorph form 1 is formed.

These data suggest, that the preparation of the polymorph form 1 is straightforward. In case of using seeding crystal of the polymorph form 1, the expected result should be the precipitation of the polymorph form 1 also.

Despite of the teaching written above, we have found, that in case of using either acetone or a lot of different solvents or mixtures of them, the polymorph form 1 has not been formed either the polymorph crystal form 1 has been used as seeding crystal, or not.

Table 1 demonstrates that in the case of the precipitation of the solution of clopidogrel hydrogensulfate in an organic solvent by salt formation, the more stable polymorph 2 form is obtained.

The following examples below are carried out with the addition of 96w/w% sulfuric acid to a solution of clopidogrel base in about an equimolar amount.

**Table 1.**

| **Experiment** | **Clopidogrel base** | **Solvent (amount)** | **T_{S}** | **Seeding crystal (amount)** | **Product morphology** |
|---|---|---|---|---|---|
| CLP-142 | 38.6g | Acetone (119 ml) | 20°C | - | polymorph 2. |
| CLP-144 | 38.6 g | Acetone (119 ml) | 20°C | polymorph 1. (0.87g) | polymorph 2. |
| CLP-130 | 38 g | Acetone (330 ml) | 5°C | polymorph 1. (0.15g) | polymorph 2. |
| CLP-188 | 27.8 g | Dichloromethane (300 ml) | 15°C | polymorph 1. (0.15g) | polymorph 2. |
| CLP-196 | 28.55g | ethyl acetate(78 ml) acetone (172 ml) mixture | 15°C | polymorph 1. (0.15g) | polymorph 2. |
| CLP-201 | 28 g | Dichloromethane (200ml)- acetone (119 ml) mixture | 15°C | polymorph 1. (0.15g) | polymorph 2. |
| CLP-208 | 36.9 | Methylethylketone (300 ml) - acetone (119 ml) mixture | 15°C | polymorph (0.15g) | 1. polymorph 2. |

T_{S} The temperature of the reaction mixture during the addition of sulfuric acid.

Detailed specification of one of these examples above is described in the experimental section as comparative example "A".

According to the specification of the International Patent Application No. 03/051362, the kinetically controlled polymorph form 1 can be produced by modifying the polarity of the organic solution containing clopidogrel hydrogensulfate, which reduce the solubility of the product. Our experiments demonstrate that even if the suggested ether type solvents are used as " B " type solvents, amorphous form is obtained instead of the expected polymorph form 1.

In the examples written below, preparation of the produce is carried out by adding 96 w/w % sulfuric acid to the solution of clopidogrel bass in about an equimolar amount.

**Table 2**

| **Example** | **Clopidogrel base** | **"A"type solvent** | **T_{S}** | **"B" type solvent** | **Product morphology** |
|---|---|---|---|---|---|
| Amorphous 1* | 32,2g | Acetone | 10-15°C | Diisopropyl ether | Amorphous |
| Amorphous 2* | 32,2g | Dichloromethan | 10-15°C | Diisopropyl ether | Amorphous |
| Amorphous 3** | 32,2g | mixture of 2-propanol and diisopropyl ether | 10-15°C | Diisopropyl ether | Amorphous |

| | | | | | |
|---|---|---|---|---|---|
| T_{S}: The temperature of the reaction mixture during addition of sulfuric acid. * Salt formation is carried out in a solution of clopidogrel base in an "A" type solvent. ** Solution of the clopidogrel base in an "A" type solvent is mixed with a mixture of "B" type solvent and sulfuric acid. | | | | | |

Detailed specification of one of these examples above is described in the experimental section as comparative example "B".

If the solvent "B" contains seeding crystals of the polymorph 1, the clopidogrel hydrogensulfate is formed as polymorph form 1.
According to our invention the polymorph form 1 can be prepared reproducibly using different types of solvents as solvent type "A" or "B ".

X-ray diffraction data of the clopidogrel hydrogensulfate having polymorph form 1 prepared according to present invention are summarised in the Table 3. Measurement conditions were as follows:

| | |
|---|---|
| Equipment: | BRUKER D8 ADVANCED |
| Radiation: | CuKα₁(λ=1.54060 Å), CuKα₂(λ=1.54439Å) |
| Voltage: | 40 kV |
| Zero-signal current: | 30 mA |
| Accessories: | Gödel mirror |
| | Soller slot |
| Used standard reference: | SRM675 |
| Mica Powder (synthetic florophlogapite), serial number: | 981307. |
| Continuous measurement Θ/ Θ scan: | 5 - 35.00° 2 Θ |
| Step scale: | 0.04° |
| Sample: | flat surface, unpulverised , stored and measured at room temperature. |

**Table 3 Diffraction lines and their relative intensity (>5%)**

| Peak | 2*th | D(hkl) | I(abs) | I(rel) |
|---|---|---|---|---|
| No. | [deg] | [L] | [cps] | [%] |
| 1 | 9.19 | 9.6216 | 241 | 22.6 |
| 2 | 10.87 | 8.1339 | 224 | 21.0 |
| 3 | 11.49 | 7.6969 | 152 | 14.2 |
| 4 | 13.80 | 6.4143 | 71 | 6.6 |
| 5 | 14.38 | 6.1532 | 137 | 12.8 |
| 6 | 14.81 | 5.9772 | 198 | 18.5 |
| 7 | 15.24 | 5.8083 | 156 | 14.6 |
| 8 | 15.49 | 5.7169 | 193 | 18.1 |
| 9 | 16.32 | 5.4285 | 53 | 5.0 |
| 10 | 17.95 | 4.9386 | 121 | 11.3 |
| 11 | 18.28 | 4.8498 | 103 | 9.7 |
| 12 | 18.49 | 4.7940 | 133 | 12.5 |
| 13 | 18.97 | 4.6758 | 170 | 15.9 |
| 14 | 19.65 | 4.5136 | 119 | 11.1 |
| 15 | 20.54 | 4.3203 | 315 | 29.5 |
| 16 | 21.59 | 4.1127 | 130 | 12.2 |
| 17 | 21.87 | 4.0614 | 143 | 13.4 |
| 18 | 22.60 | 3.9308 | 93 | 8.7 |
| 19 | 23.17 | 3.8357 | 1068 | 100 |
| 20 | 23.43 | 3.7937 | 173 | 16.2 |
| 21 | 23.84 | 3.7294 | 196 | 18.4 |
| 22 | 24.41 | 3.6434 | 73 | 6.9 |
| 23 | 25.52 | 3.4875 | 356 | 33.3 |
| 24 | 25.95 | 3.4314 | 100 | 9.3 |
| 25 | 26.54 | 3.3553 | 96 | 9.0 |
| 26 | 27.35 | 3.2587 | 95 | 8.9 |
| 27 | 28.47 | 3.1323 | 83 | 7.8 |
| 28 | 28.92 | 3.0849 | 165 | 15:4 |
| 29 | 30.76 | 2.9043 | 131 | 12.3 |
| 30 | 32.64 | 2.7412 | 57 | 5.3 |
| 31 | 32.94 | 2.7172 | 73 | 6.9 |

Great advantage of the present invention is that the used solvents can be chosen from more types of solvents, than it is known from the state of the art, and the chosen solvents can be adapted easily to the used technology for the production of polymorph form 1 of clopidogrel hydrogensulfate in a reproducible way.
For example, use of dichloromethane as "A" type solvent is very advantageous because it can be used for the extraction of clopidogrel base obtained when setting it free from its camphorsulfonic acid salt. According to the present invention, clopidogrel hydrogensulfate can be obtained as polymorph form 1 in one step without exchange of the solvent. Thus, the required time and costs of chemicals are reduced as well.

Further details are described below without the limitation of the scope of the present invention to the examples.

### Example 1

### Clopidogrel hydrogensulfate polymorph 1

A solution containing 32.2 g of clopidogrel base in 130 ml of acetone is stirred and cooled to 10-15°C then 10.2 g of 96 w/w% sulfuric acid are added. The obtained mixture is added to the suspension of 10 g of clopidogrel hydrogensulfate polymorph form 1 in 1000 ml of diisopropyl ether dropwise at 0°C in 15-20 minutes under stirring. The reaction mixture is stirred for an additional hour at 0°C, filtered, the precipitate is washed with 2x100 ml of cold diisopropyl ether, dried at 50°C for five days.
Thus, 48 g (90.5%) of clopidogrel hydrogensulfate polymorph form 1 are obtained. The melting point of the product is 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4.H), 3.74 (s, 3H), 3.08 (b, 2H).

¹³C-NMR:167.65 134.38,132.07, 131.89, 130.74, 128.46,125.67, 124.92, 65.77, 53.57, 50.27, 48.86, 22.61

### Example 2

### Clopidogrel hydrogensulfate polymorph 1

A solution containing 32.2 g of clopidogrel base in 200 ml of dichloromethane is stirred and cooled to 0°C, then 9.7 g of 96 w/w% sulfuric acid are added. The mixture is added to the suspension of 10 g of clopidogrel hydrogensulfate polymorph form 1 in 850 ml of diisopropyl ether dropwise at 0°C in 15-20 minutes under stirring. The reaction mixture is stirred for an additional hour at 0°C, filtered, the precipitate is washed with 2x100 ml of cold diisopropyl ether, then dried for five days at room temperature. Thus, 47 g (88.1 %) of clopidogrel hydrogensulfate polymorph form 1 are obtained. The melting point of the product is 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4H), 3.74 (s, 3H), 3.08 (b, 2H).

¹³C-NMR: 167.65, 134.38, 132.07, 131.89, 130.74, 128.46, 125.67, 124.92, 65.77, 53.57, 50.27, 48.86, 22.61.

### Example 3

### Clopidogrel hydrogensulfate polymorph 1

A solution containing 32.2 g of clopidogrel base in 140 ml of 2-propanol is stirred and cooled between 10-15°C then 10.2 g of 96 w/w% sulfuric acid are added. The mixture is added to the suspension of 10 g of clopidogrel hydrogensulfate polymorph form 1 in 850 ml of diisopropyl ether dropwise at 0°C in 15-20 minutes under stirring. The reaction mixture is stirred for an additional hour at 0°C, filtered, the precipitate is washed with 2x100 ml of cold diisopropyl ether, then dried for five days at room temperature. Thus, 49 g (92.8%) of clopidogrel hydrogensulfate polymorph form 1 are obtained. The melting point of the product is 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4H), 3.74 (s, 3H), 3.08 (b, 2H).

¹³C-NMR: 167.65, 134.38, 132.07, 131.89, 130.74, 128.46, 125.67, 124.92, 65.77, 53.57, 5.0.27, 48.86, 22.61.

### Example 4

### Clopidogrel hydrogensulfate polymorph 1

To a solution containing 32.2 g of clopidogrel base in a mixture of 860 ml if diisopropyl ether and 140 ml of 2-propanol 10 g clopidogrel hydrogensulfate polymorph form 1 are added. The suspension is stirred and cooled to 0°C, then a mixture of 50 ml of diisopropyl ether and 10.2 g of 96 w/w% sulfuric acid are added dropwise in 15-20 minutes under stirring. Then the reaction mixture is stirred for an additional hour at 0°C, filtered, the precipitate is washed with 2x100 ml of cold diisopropyl ether, then dried for five days at room temperature
Thus, 4.9 g (92 8%) of clopidogrel hydrogensulfate polymorph form 1 are obtained. The melting point of the product are 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4H), 3.74 (s, 3H), 3.08(b,2H).

¹³C-N-NM: 167.65, 134.38,132.07, 131.89,130.74, 128.46,125.67, 124.92, 65.77, 53-57, 50.27,48.86,22.61.

### Example 5

### Clopidogrel hydrogensulfate polymorph 1

A solution containing 32.2 g of clopidogrel base in 200 ml of dichloromethane is stirred and cooled to 0°C, then 9.7 g of 96 w/w% sulfuric acid are added. The mixture is added to the suspension of 10 g of clopidogrel hydrogensulfate polymorph form 1 in 850 ml of cyclohexane dropwise at 8-10°C in 15-20 minutes under stirring. Then the reaction mixture is stirred for an additional hour at 8-10°C, filtered the precipitate is washed with 2x100 ml of cold cyclohexane, then dried for five days at room temperature.
Thus, 49 g (92.8%) of clopidogrel hydrogensulfate polymorph form 1 are obtained. The melting point of the product is 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4H), 3.74 (s, 3H), 3.08 (b, 2H).

¹³C-NMR: 167.65, 134.38,132.07, 131.89, 130.74, 128.46, 125.67, 124.92, 65.77, 53.57, 50.27, 48.86, 22.61.

### Example 6

### Clopidogrel hydrogensulfate polymorph 1

A solution containing 32.2 g of clopidogrel base in 200 ml of dichloromethane is stirred and cooled to 0°C, then 10.2 g of 96 w/w% sulfuric acid are added. The mixture is added to the suspension of 10 g of clopidogrel hydrogensulfate polymorph form 1 in 1000 ml of ethyl acetate dropwise at 20°C under stirring in 30 minutes. Then the reaction mixture is stirred for additional 15 minutes, filtered, the precipitate is washed with 2x100 ml of cold ethyl acetate, then dried.
Thus, 44.5 g (82%) of clopidogrel hydrogensulfate polymorph form 1 are obtained. The melting point of the product is 184°C.

¹H-NMR (DMSO-d₆, i400): 7.88 (d, J=6.5 Hz, 1H), 7.64 (dd, J1=1.8 Hz, J1=7.9 Hz 1H), 7.52 (m, 2H), 7.42 (d, J=5.1 Hz, 1H), 6.87 (d, J=5.1 Hz, 1H), 5.57 (b, 1H), 4.20 (b, 4H), 3.74 (s, 3H), 3.08 (b, 2H).

¹³C-,N.MR: 167.65, 134.38, 132.07, 131.89, 130.74, 128.46, 125.67, 124.92, 65.77, 53.57, 50.27, 48.86, 22.61.

### Comparative examples

### Comparative example "A"

### Clopidogrel hydrogensulfate polymorph 2

### (CLP-144)

A solution of 38.6 g of clopidogrel base in 119 ml acetone is filled into a 500 ml SCHMIZO type duplicator equipped with a variable-speed anchor-type agitator. A LAUDA RE-306 type programmable thermostat is connected to the duplicator to keep the desired temperature, or to accomplish a cooling or heating program. The temperature of the solution is adjusted to 6°C with the thermostat. After addition of 0.9 g of clopidogrel hydrogensulfate polymorph form 1 to the solution, 6 ml of concentrated sulfuric acid are added in 5 minutes while the temperature of the reaction mixture is kept under 20°C. The crystalline suspension is stirred for additional 4.5 hours at 5 °C, the precipitate is filtered, washed with cold acetone and dried for 24 hours at 40 °C.
Thus, 40.09g (80%) of clopidogrel hydrogensulfate polymorph form 2 are obtained.

### Comparative example "B"

### Clopidogrel hydrogensulfate amorphous form

A solution containing 32.2 g of clopidogrel base in 140 ml of dichloromethane is stirred and cooled to between 10-15°C, then, 10.2 g of 96 w/w% sulfuric acid are added. The mixture is added to 850 ml of diisopropyl ether dropwise at 0°C in 15-20 minutes under stirring. Then the reaction mixture is stirred for an additional hour at 0°C, filtered, the precipitate is washed with 2x100 ml of cold diisopropyl ether.
Thus, 39 g (92.8%) of clopidogrel hydrogensulfate amorphous form are obtained.

## Claims

1. Process for the preparation of the polymorph form 1 of methyl (S)-(+)-(2-chlorophenyl)-2-(6,7-dihydro-4H-thieno[3,2-c]pyridine-5-yl-acetate hydrogensulfate of the formula 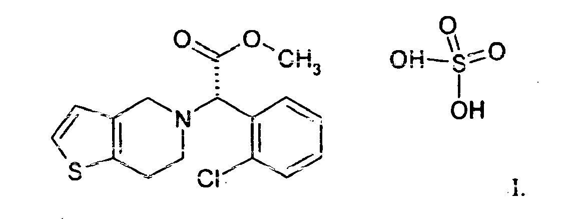 which comprises
a.) dissolving clopidogrel base in a solvent selected from a halogenated solvent, a ketone or 2-propanol, adding sulfuric acid or a mixture of sulfuric acid and a solvent selected from a halogenated solvent, a ketone, a protic solvent, or a solvent selected from an ether type solvent, a saturated alkyl hydrocarbon, an ester type solvent, the obtained mixture containing clopidogrel hydrogensulfate is added to a mixture containing a solvent selected from an ether type solvent, a saturated alkyl hydrocarbon, or an ester type solvent, and clopidogrel hydrogensulfate polymorph form I as a suspension,
or
b.) dissolving clopidogrel base in a mixture of solvents selected from a halogenated solvent, a ketone or 2-propanol, and a solvent selected from an ether type solvent, a saturated alkyl hydrocarbon, ester type solvent, clopidogrel hydrogensulfate polymorph form 1 is added to the solution, then adding sulfuric acid or a mixture of sulfuric acid with a a solvent selected from a halogenated solvent, a ketone or a protic solvent, or a solvent selected from an ether type solvent, a saturated alkyl hydrocarbon, ester type solvent to the obtained mixture,
and
filtering, optionally washing and drying the formed precipitate.

2. Process according to Claim 1 which comprises using preferably chlorinated solvents, more preferably dichloromethane as halogenated solvents, preferably acetone as ketone.

3. Process according to Claim 1-2 which comprises using diethyl ether, tetrahydrofurane or diisopropyl ether, preferably diisopropyl ether as ether type solvent.

4. Process according to Claim 1-3 which comprises using saturated alkyl hydrocarbons preferably cyclohexane, hexane or heptane more preferably cyclohexane as saturated alkyl hydrocarbon.

5. Process according to Claim 1-4 which comprises using ethyl acetate as an ester type solvent.

6. Process according to Claim 1 which comprises dissolving the clopidogrel base in dichloromethane, the obtained mixture is cooled to 0°C under stirring, adding 96 w/w% of sulfuric acid to the solution, adding the obtained mixture to a suspension of clopidogre1 hydrogensulfate of the polymorph 1 form in cyclohexane at 8-10°C, then filtering, drying the obtained precipitate

7. Process according to Claim 1 which comprises dissolving the clopidogrel base in dichloromethane, the obtained mixture is cooled to 0°C under stirring, adding 96 w/w% of sulfuric acid to the solution, adding the obtained mixture to a suspension of clopidogrel hydrogensulfate of the polymorph 1 form in ethyl acetate at 20°C, then filtering, drying the obtained precipitate.

## Patentansprüche

1. Verfahren zur Herstellung der polymorphen Form 1 von Methyl (S)-(+)-(2-chlorphenyl)-2-(6,7-dihydro-4H-thieno[3,2-c]pyridin-5-yl-acetathydrogensulfat der Formel 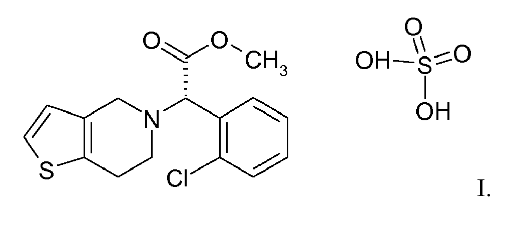 umfassend
a.) Das Lösen einer Clopidogrelbase in einem Lösungsmittel ausgewählt aus einem halogenierten Lösungsmittel, einem Keton oder einem 2-Propanol, das Hinzufügen von Schwefelsäure oder einer Mischung von Schwefelsäure und einem Lösungsmittel ausgewählt aus einem halogenierten Lösungsmittel, einem Keton, einem protischen Lösungsmittel, oder einem Lösungsmittel ausgewählt aus einem Lösungsmittel vom Ethertyp, einem gesättigten Alkylkohlenwasserstoff, einem Lösungsmittel vom Estertyp, die erhaltene Mischung enthaltend Clopidogrelhydrogensulfat wird einer Mischung umfassend ein Lösungsmittel ausgewählt aus einem Lösungsmittel vom Ethertyp, einem gesättigten Alkylkohlenwasserstoff, oder einem Lösungsmittel vom Estertyp, und der polymorphen Form I von Clopidogrelhydrogensulfat zugefügt,
oder
b.) Das Lösen von Clopidogrelbase in einer Lösungsmittelmischung ausgewählt aus einem halogenierten Lösungsmittel, einem Keton oder 2-Propanol, und einem Lösungsmittel ausgewählt aus einem Lösungsmittel vom Ethertyp, einem gesättigten Alkylkohlenwasserstoff, einem Lösungsmittel vom Estertyp, die polymorphe Form I von Clopidogrelhydrogensulfat wird der Lösung hinzugefügt, das Hinzufügen von Schwefelsäure oder einer Mischung aus Schwefelsäure mit einem Lösungsmittel ausgewählt aus einem halogenierten Lösungsmittel, einem Keton, einem protischen Lösungsmittel oder einem Lösungsmittel ausgewählt aus einem Lösungsmittel vom Etheryp, einem gesättigten Alkylkohlenwasserstoff, einem Lösungsmittel vom Estertyp zu der erhaltenen Mischung,
und
dem Filtern, optional Waschen und Trocknen des gebildeten Präzipitats.

2. Verfahren nach Anspruch 1, umfassend den Gebrauch von vorzugsweise chlorierten Lösungsmitteln, noch bevorzugter von Dichlormethan als halogenierten Lösungsmitteln, vorzugsweise von Aceton als Keton.

3. Verfahren nach Anspruch 1-2 umfassend den Gebrauch eines Diethylether, Tetrahydrofuran oder Diisopropylether, vorzugsweise Diisopropylether als Lösungsmittel vom Etheryp.

4. Verfahren nach Anspruch 1-3 umfassend den Gebrauch von gesättigten Alkylkohlenwasserstoffen vorzugsweise Cyclohexan, Hexan oder Heptan, noch bevorzugter Cyclohexan als gesättigtem Alkylkohlenwasserstoff.

5. Verfahren nach Anspruch 1-4 umfassend den Gebrauch von Ethylacetat als einem Lösungsmittel vom Esteryp.

6. Verfahren nach Anspruch 1 umfassend das Lösen der Clopidogrelbase in Dichloromethan, die erhaltene Mischung wird auf 0°C unter Rühren abgekühlt, das Hinzufügen von 96 w/w% Schwefelsäure zu der Lösung, das Hinzufügen desrerhaltenen Mischung zu einer Suspension der polymorphen Form I von Clopidogrelhydrogensulfat in Cyclohexan bei 8-10°C, dann Filtern, Trocknen des erhaltenen Präzipitats.

7. Verfahren nach Anspruch 1, umfassend das Lösen der Clopidogrelbase in Dichloromethan, die erhaltene Mischung wird auf 0°C abgekühlt unter Rühren, das Hinzufügen von 96 w/w% Schwefelsäure zu der Lösung, das Hinzufügen der erhaltenen Mischung zu einer Suspension der polymorphen Form I von Clopidogrelhydrogensulfat in Ethylacetat bei 20°C, dann Filtern, Trocknen des erhaltenen Präzipitats.

## Revendications

1. Procédé pour la préparation de la forme polymorphe 1 de l'hydrogénosulfate de l'acétate méthylique du (S)-(+)-(2chlorophényl)-2-(6,7-dihydro-4H-thiéno-[3,2-c]-pyridine-5-yl de formule: 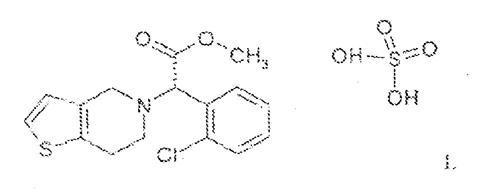 qui comprend
a.) la dissolution du clopidogrel base dans un solvant choisi dans le groupe comprenant les solvants halogénés, les cétones ou le 2-propanol, l'addition d'acide sulfurique ou d'un mélange d'acide sulfurique et d'un solvant choisi dans le groupe comprenant les solvants halogénés, les cétones, les solvants protiques, ou d'un solvant choisi dans le groupe comprenant les solvants de type éther, les hydrocarbures alkyliques saturés, les solvants de type esters, le mélange obtenu contenant de l'hydrogénosulfate de clopidogrel étant ajouté à un mélange contenant un solvant choisi dans le groupe comprenant les solvants de type éther, les hydrocarbures alkyliques saturés ou les solvants de type esters et la forme polymorphe 1 du clopidogrel hydrogénosulfate sous forme de suspension,
ou
b.) la dissolution du clopidogrel base dans un mélange de solvants choisi dans le groupe comprenant les solvants halogénés, les cétones ou le 2-propanol, et d'un solvant choisi dans le groupe comprenant les solvants de type éthers, les hydrocarbures alkyliques saturés, les solvants de type esters, l'addition à la solution de la forme polymorphe 1 de l'hydrogénosulfate de clopidogrel, puis l'addition au mélange obtenu d'acide sulfurique ou d'un mélange d'acide sulfurique avec un solvant choisi dans le groupe comprenant les solvants halogénés, les cétones ou les solvants protiques, ou d'un solvant choisi dans le groupe comprenant les solvants de type éthers, les hydrocarbures alkyliques saturés et les solvants de type esters,
et
la filtration, éventuellement le lavage et le séchage du précipité formé.

2. Procédé selon la revendication 1, qui comprend l'utilisation de préférence de solvants chlorés, plus particulièrement de dichlorométhane en tant que solvant halogéné, de préférence d'acétone en tant que cétone.

3. Procédé selon la revendication 1-2 qui comprend l'utilisation de diéthyle éther, tétrahydrofurane ou diisopropyle éther, de préférence de diisopropyle éther en tant que solvant de type éther.

4. Procédé selon la revendication 1-3, qui comprend l'utilisation d'hydrocarbures alkyliques saturés, de préférence de cyclohexane, d'hexane ou d'heptane, plus particulièrement de cyclohexane en tant qu'hydrocarbures alkyliques saturés.

5. Procédé selon la revendication 1-4, qui comprend l'emploi d'acétate d'éthyle en tant que solvants de type ester.

6. Procédé selon la revendication 1, qui comprend la dissolution du clopidogrel base dans le dichlorométhane, le refroidissement à 0°C et sous agitation du mélange obtenu, l'addition à la solution d'acide sulfurique à 96 p/p%, l'addition du mélange obtenu à une suspension de la forme polymorphe 1 d'hydrogénosulfate de clopidogrel dans du cyclohexane à 8-10°C, puis la filtration et le séchage du précipité obtenu.

7. Procédé selon la revendication 1, qui comprend la dissolution du clopidogrel base dans du dichlorométhane, le refroidissement à 0°C et sous agitation du mélange obtenu, l'addition d'acide sulfurique à 96 p/p% à la solution, l'addition du mélange obtenu à une suspension de la forme polymorphe 1 d'hydrogénosulfate de clopidogrel dans l'acétate d'éthyle à 20°C, puis la filtration et le séchage du précipité obtenu.
